# EUROPEAN PATENT SPECIFICATION

(11) **EP 2 456 881 B1**
(45) Date of publication and mention of the grant of the patent: **29.04.2020**
(21) Application number: 10802787.1
(22) Date of filing: 20.07.2010
(51) Int. Cl.: C12Q 1/02, G01N 33/483, C12M 1/34, G01N 15/14, G01N 15/10

(54) **METHOD AND APPARATUS FOR PERFORMING CYTOMETRY**
VERFAHREN UND VORRICHTUNG ZUR DURCHFÜHRUNG EINER ZYTOMETRIE
PROCÉDÉ ET APPAREIL POUR EFFECTUER UNE CYTOMÉTRIE

(30) Priority: 21.07.2009 US 227397 P; 04.12.2009 US 283542 P; 01.03.2010 US 660570
(43) Date of publication of application: 30.05.2012
(73) Proprietor: Sony Corporation, Tokyo 108-0075 (JP); Sony Corporation Of America, New York, NY 10022-3211 (US)
(72) Inventor: SHINODA, Masataka, Atsugi-shi Kanagawa 243-0014 (JP); DURACK, Gary, Urbana Illinois 61802 (US)
(74) Representative: D Young & Co LLP
(86) International application number: PCT/US2010/042629
(87) International publication number: WO 2011/011430

(56) References cited:
- EP-A2- 2 053 380
- CA-A1- 2 540 474
- CA-A1- 2 684 612
- US-A1- 2005 243 304
- US-A1- 2005 243 304
- US-A1- 2006 020 371
- US-A1- 2006 281 143
- US-A1- 2008 286 751
- WOLFF, A. ET AL. LAP CHIP. vol. 3, 23 January 2003, pages 22 - 27, XP009045495
- LI, W. C. ET AL. MOLECULAR & CELLULAR PROTEOMICS. vol. 5, 06 April 2006, pages 1261 - 1273, XP008150264

## Description

### BACKGROUND

### Field of the invention

Embodiments of the present invention generally relate to flow cytometry and, more particularly, to a method and apparatus for performing cytometry using a microfluidic assembly comprising a holder and a cytometry cell.

### Description of the Related Art

Flow cytometry based cell sorting is widely used in various life science researches (e.g., genetics, immunology, molecular biology, environmental science and/or the like). The flow cytometry based cell sorting facilitates measurement, classification and/or sorting of the cells. Such sorting can be performed at a rate of several thousand cells per second. The flow based cell sorting may be utilized to extract highly pure sub-population of the cells from a heterogeneous cell sample.

A flow cytometry system comprises a microfluidic chip used to analyze the cells in a sample fluid as well as create sample fluid droplets to facilitate cell sorting. The microfluidic chip controls the flow of sample fluid through a microfluidic channel within which the cells within the sample fluid can be optically analyzed. A piezoelectric transducer is coupled to a distal end of the microfluidic channel to create droplets. The microfluidic chip also comprises an electrode coupled to the sample fluid to facilitate electrically charging the cells within the fluid. The charged droplets are then electrostatically sorted within an electrostatic sorting assembly.

Generally, to facilitate cell analysis, the cells within the sample fluid flowing through the microfluidic channel are exposed to a focused, high intensity beam (e.g., a laser beam and/or the like). As the cells pass through the focused, high intensity beam, each cell is illuminated and therefore, emits a fluorescent light. A computer is utilized to monitor and analyze emission intensities of each cell and produce a result based on the analysis. The data is then utilized to classify the each cell for sorting. Such flow cytometry based cell sorting facilitates analysis in a wide range of applications such as isolation of rare population of immune system cells for AIDS research, generation of atypical cells for cancer research, isolation of specific chromosomes for genetic studies, isolation of various species of microorganisms for environmental studies and/or the like. For example, adult stem cell may be isolated from bone marrow and may be re-infused back to a patient.

Currently, droplet cell sorters are widely used for sorting the cells after optical analysis. The droplet cell sorter utilizes micro droplets as containers to sort selected cells to a collection vessel. The micro droplets are formed by coupling ultrasonic energy to the fluid sample stream within the microfluidic channel. The droplets including the selected cells are then electrostatically steered to the collection vessel. Such sorting process allows a significant number of the cells to be sorted in a relatively short period of time.

In a typical cytometry system, the samples are collected at one location and transported to the location of the cytometry system. The samples are then transferred to a sample reservoir that is coupled to the cytometry chip. The cytometry chip is an info complement of the cytometry system. After each use, substantial effort is used to clean and sterilize the cytometry chip such that processing of a new sample is not contaminated by prior sample.

Therefore, there is a need in the art for a method and apparatus for performing cytometry using a microfluidic assembly that provides a low probability of sample contamination.

US 2008/286751 A1 discloses a dispensing device for droplets comprising means for measuring a physical property of a fluid flow in a channel, the device having inputs and outlets for the fluid and which may be within a box or mould.

US 2005/243304 A1 discloses methods and apparatus for receiving a removable media member providing tighter alignment tolerances between an inserted removable media member and a receiving device. The removable media member may include one or more fluid ports and may be a fluidic cartridge adapted for use in flow cytometry.

CA 2684612 A1 discloses the packaging of a microfluidic channel network in a self-contained plastic cartridge that enables an interconnection to a macro-scale instrument.

CA 2540474 A1 discloses a real-time digital cytometer on a chip system utilizing a custom near field CMOS active pixel intelligent sensor that is flip-chip attached to a fluidic microchannel etched in a thin glass substrate.

### SUMMARY OF THE INVENTION

Embodiments of the present disclosure generally include a method and apparatus for performing cytometry and are defined by the appended claims.

### BRIEF DESCRIPTION OF THE DRAWINGS

So that the manner in which the above recited features of the present invention can be understood in detail, a more particular description of the invention, briefly summarized above, may be had by reference to embodiments, some of which are illustrated in the appended drawings. It is to be noted, however, that the appended drawings illustrate only typical embodiments of this invention and are therefore not to be considered limiting of its scope, for the invention may admit to other equally effective embodiments.
Figure 1 is a simplified schematic, block diagram of a cytometry system, according to one or more embodiments of the present invention;
Figure 2 is a block diagram of a top plan view of a microfluidic assembly, according to one or more embodiments of the present invention;
Figure 3 depicts a side view of a microfluidic assembly, according to one or more embodiments of the present invention;
Figure 4 depicts a horizontal cross section of a cytometry chip, according to one or more embodiments of the present invention;
Figure 5 depicts a vertical cross section of a cytometry chip, according to one or more embodiments of the present invention;
Figure 6 depicts a detailed cross section of a microfluidic assembly, according to one or more embodiments of the present invention;
Figure 7 depicts a cross section of a holder, according to one or more embodiments of the present invention;
Figure 8 depicts a perspective view of an alternative embodiment of a microfluidic assembly comprising a holder for a cytometry chip;
Figure 9 depicts a cross-sectional view of an alternative embodiment of a microfluidic assembly comprising a holder for a cytometry chip;
Figure 10 depicts a horizontal cross section of an alternative embodiment of a cytometry chip, according to one or more embodiments of the present invention;
Figure 11 depicts a vertical cross section of an alternative embodiment of the cytometry chip in Figure 10, according to one or more embodiments of the present invention.

### DETAILED DESCRIPTION

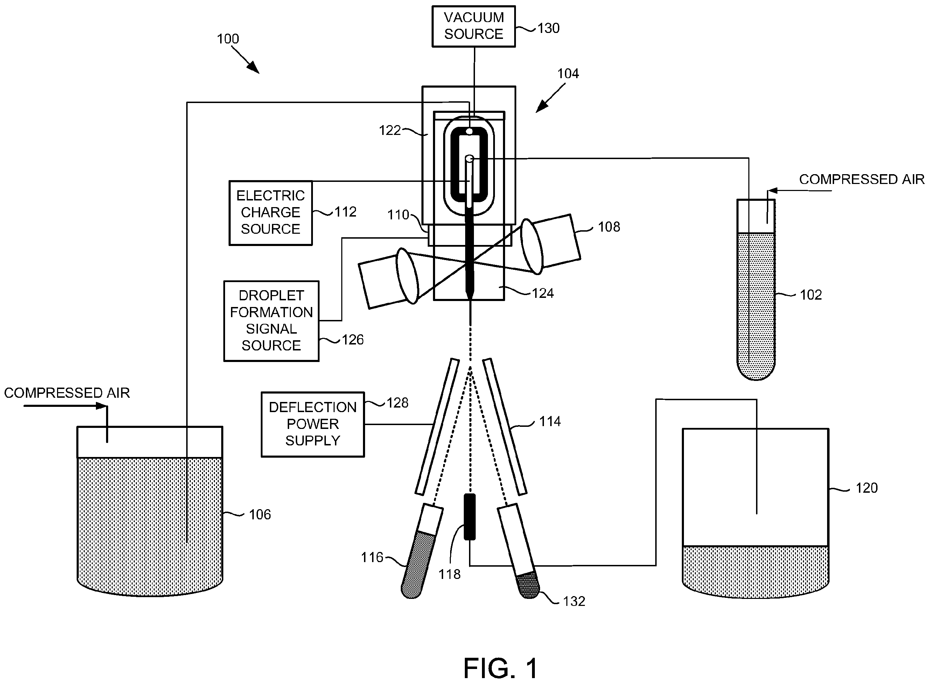

Figure 1 is a simplified schematic, block diagram of a system 100 for performing cytometry according to one or more embodiments of the present invention. The system 100 includes a sample vessel 102, a microfludic assembly 104, a sheath vessel 106, an optics module 108, a piezoelectric transducer 110, and an electric charge source 112, where each is coupled to the microfluidic assembly 104. The system 100 further comprises one or more deflection plates 114, a target sample vessel 116 and a waste vessel 118 coupled to a waste collection vessel 120.

In one embodiment, the sample vessel 102 provides a sample fluid containing cells to be analyzed and/or sorted, to the microfluidic assembly 104. According to various embodiments, the microfluidic assembly 104 comprises a holder 122 and a cytometry chip 124. The holder 122 provides an interface between the cytometry chip 124 and the sample vessel, electric charge source, and the sheath vessel. The cytometry chip 124 is configured to provide a three dimensional

(3D) laminar flow of the sample fluid. In one or more embodiments, the cytometry chip 124 is injection molded. In another embodiment, the cytometry chip 124 comprises industrial plastic such as a Cyclo Olefin Polymer (COP) material, or other plastic. As a result, the cytometry chip 124 is transparent such that the optics module 108 can analyze the sample fluid stream as described further below. In one embodiment, the microfluidic assembly 104 is disposable.

According to one or more embodiments, the sheath vessel 106 includes a sheath fluid that facilitates creating a steady flow of the sample through the cytometry chip 124. The sheath fluid permits cells in the sample fluid to be drawn into a single file line, as discussed further below. The sheath fluid is collected along with the sorted cells by the system 100 and therefore forms part of the post-sort environment for performing cytometry. Thus, it is desirable that the sheath fluid provides a protective effect to the cells upon contact with the cells. In one embodiment, the sheath fluid comprises a buffer or buffered solution. For example, the sheath fluid comprises 0.96% Dulbecco's phosphate buffered saline (w/v), 0.1 % BSA (w/v), in water at a pH of about 7.0.

According to various embodiments, the microfluidic assembly104 comprise a vacuum port that is coupled to a vacuum source 130. The vacuum is supplied to the microfluidic assembly 104 to facilitate extraction of fluids from the assembly 104when clog occurs in a channel of the cytometry chip 124.

According to various embodiments, the optics module 108 includes one or more optical lenses for focusing a beam of electromagnetic radiation on the fluid stream at a region proximate an output of the microfluidic assembly 104. Thus, the described embodiment forms a so-called flow-cell system. In one embodiment, the optics module 108 is configured to focus a beam of electromagnetic radiation (e.g., a laser light) on the fluid stream as a beam spot, so that the cells to be analyzed pass through the spot. The beam may be laser light in the visible or ultraviolet portion of the spectrum, for example, having a wavelength of about 300-1000 nm, although other wavelengths may be used. The wavelength of the laser light may be selected so that it is capable of exciting a particular fluorochrome used to analyze the cells.

In one or more embodiments, the piezoelectric transducer 110 is coupled to a signal source 126 such that energy can be delivered to the fluid stream at a frequency that causes the fluid stream to break into droplets. In one embodiment, the piezoelectric transducer 110 is configured to operate at the frequency of ten Kilo Hertz (10 KHz). In one embodiment, the piezoelectric transducer 110 is coupled to the microfluidic assembly 104 to create droplets containing the individual sample cells as described further below.

According to one or more embodiments, the electric charge source 112 is configured to provide an electrostatic charge to the sheath fluid. The electric charge source 112 (e.g., a 20 volt power supply circuit) is configured to charge or not charge the fluid stream prior to the formation of the droplets. The droplets carry the same charge as the fluid stream at the instant the droplet breaks from the fluid stream. The charged or uncharged droplets then pass between the one or more deflection plates 114 and are sorted by the charge into the target sample vessel 116 and/or the waste vessel 118. While the depicted system 100 produces two groups or populations of droplets using the electrostatic sorting process, the particles may be separated into any number of populations from 1 to N by placing different charge levels and/or polarities on the droplets. The amount of charge and/or the polarity of the charge controls the amount of deflection incurred as the droplets pass the deflection plates 114. A different sample vessel can be provided to capture the droplets having various charge levels and/or polarities.

According to various embodiments, the one or more deflection plates 114 are electrostatic charged and are configured to sort the droplets into the target sample vessel 116 and the waste vessel 118 according to the charge and/or polarity. It is desirable to coat the deflection plates 114 with a dull, low-emissive coating (e.g., epoxy or paint) to limit light reflected or emitted by the deflection plates 114. In one or more embodiments, the deflection plates 114 may be charged by any suitable power supply 128. It is generally desirable for the electrical potential between the two fully charged deflection plates 114 to be in the range of 2000-4000 volts. However, the electrical potential between the deflection plates 114 may be anywhere between about 1000 and 6000 volts.

The target sample vessel 116 collects the sorted cells based on the charge and/or polarity. Similarly, the waste vessel 118 collects the waste cells from the fluid stream and transfers to the waste collection vessel 120.

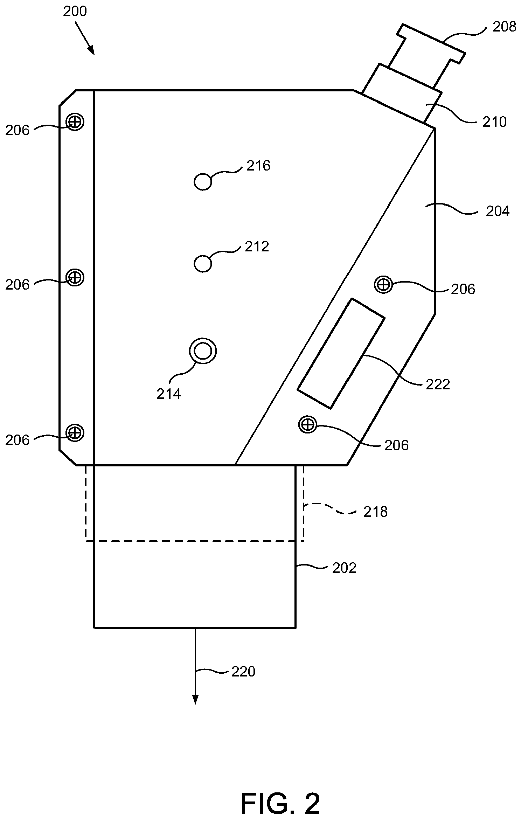

Figure 2 is a block diagram of a top plan view of a microfluidic assembly 200 according to one embodiment of the invention. The microfluidic assembly 200 comprises a cytometry chip 202 and a chip holder 204, where the chip holder 204 retains the cytometry chip 202. The chip holder 204 and the cytometry chip 202 are capable of being sterilized prior to assembly and/or after assembly to form a microfluidic assembly 200.

In one embodiment, the cytometry chip 202 is a microfluidics chip configured to sort cells. The cytometry chip 202 produces a three dimensional (3D) laminar flow of a sample fluid from a chip output 220 in the form of droplets. To facilitate droplet formation, the cytometry chip 202 is coupled to a transducer 218 that is configured to generate droplets at the chip output 220.

The chip holder 204 is configured to retain the cytometry chip 202. In one embodiment, the chip holder 204 retains the cytometry chip 202 by a pressure fit. As such, a plurality of screws 206 may be utilized to provide the pressure. The screws 206 are tightened to secure the cytometry chip 202 within the holder 204. In another embodiment, the chip holder 204 may be bonded to the chip 202 using a thermal bond, adhesive, epoxy or another bonding agent. The chip holder 204 comprises an inlet 208, a one-way valve 210, a sheath port 212, an electrode port 214 and a vacuum port 216. As is described below, the ports 212, 214 and 216 are coupled to corresponding ports on the cytometry chip 202.

According to one or more embodiments, the inlet 208 is configured to provide a sample to a sample reservoir (not shown in this view) located within the holder 204. The sample comprises the cells or other material to be sorted. The inlet 208 is coupled through the one-way valve 210 to the sample reservoir. The one-way valve 210 is configured to allow a compressed gas (e.g., air, inert gasses, and/or the like) to flow only in one direction, to apply pressure on the sample such that the sample flows through a sample port (not shown) into the cytometry chip 202. The use of a one-way valve 210 ensures the sample is not contaminated during processing, nor can the sample escape into the environment. As such, the sample may be collected in the chip holder 204, sealed from the environment, and transferred to a laboratory for further analysis and/or sorting. Accordingly, the microfluidic assembly 200 may be configured to operate in the system 100 of Figure 1. In one embodiment of the invention, the microfluidic assembly 200 is a disposable component of the system 100. As such, the sample can be collected in the field and injected into the reservoir of the holder 204, transported to the system 100 where the sample is analyzed, and then the microfluidic assembly 200 is discarded.

Upon insertion of the microfluidic assembly 200 into the system 100, the sheath port is coupled to a sheath fluid source (e.g., the sheath vessel 106 of Figure 1), the electrode port 214 is coupled to an electric charge source (e.g., the electric charge source 112 of Figure 1) and the vacuum port to 216 is coupled to a vacuum source (e.g., the vacuum source 130 of Figure 1). In one embodiment, the transducer 218 may be a component of the cytometry system 100 such that upon insertion of the microfluidic assembly 200 into the system 100 the transducer abuts the cytometry chip 202. In another embodiment, the transducer 218 may be a component of the microfluidic assembly 200. In either embodiment, the transducer 218 is configured to create droplets of the sample fluid and/or sheath fluid at the chip output 220. In one embodiment, the transducer 218 is a piezoelectric acoustic transducer configured to vibrate to generate the droplets. Once the cells are sorted, the vacuum port 216 is utilized to clean the cytometry chip 202 and the chip holder 204 prior to disposal. The vacuum port 216 is also utilized to reverse the sample flow direction if channel clogging occurs.

The holder 204 may include a means 222 for tracking the microfluidic assembly 200. One such means is a radio frequency identification tag (RFID) that is affixed to the assembly 200 and can be remotely scanned to identify and track the assembly 200. Another such means that can be remotely scanned is a barcode. A tracking means that requires contact to extract information to identify the assembly is a solid state memory comprising a serial number and/or process/sample history information.

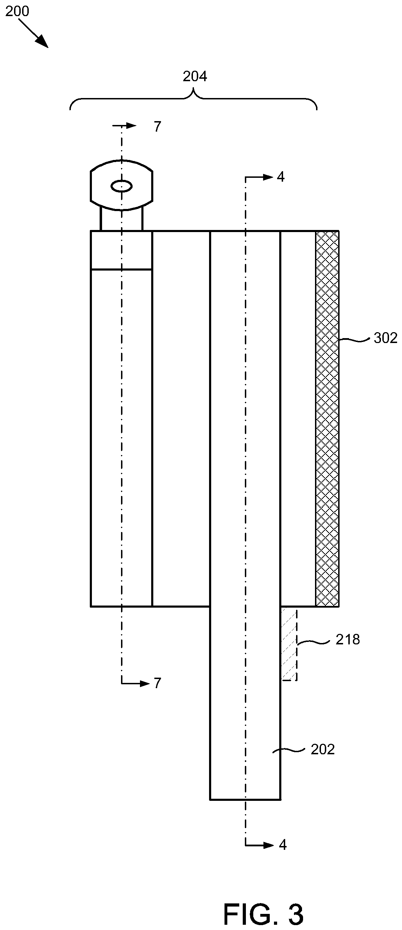

Figure 3 depicts a side view of a microfluidic assembly 200 according to various embodiments of the present invention. The microfluidic assembly 200 comprises a cytometry chip 202, a chip holder 204 and an optional temperature controller 302.

In the depicted embodiment, the chip holder 204 comprises a optional temperature controller 302. The temperature controller 302 is physically coupled a bottom side of the chip holder 204. In other embodiments, the temperature controller 302 may be coupled to another portion of the holder 204. The temperature controller 302 (e.g., a peltier temperature controller) is configured to maintain the sample at a specific temperature. In one embodiment of the invention, the temperature of the sample is maintained at a level such that cells within the sample are kept alive.

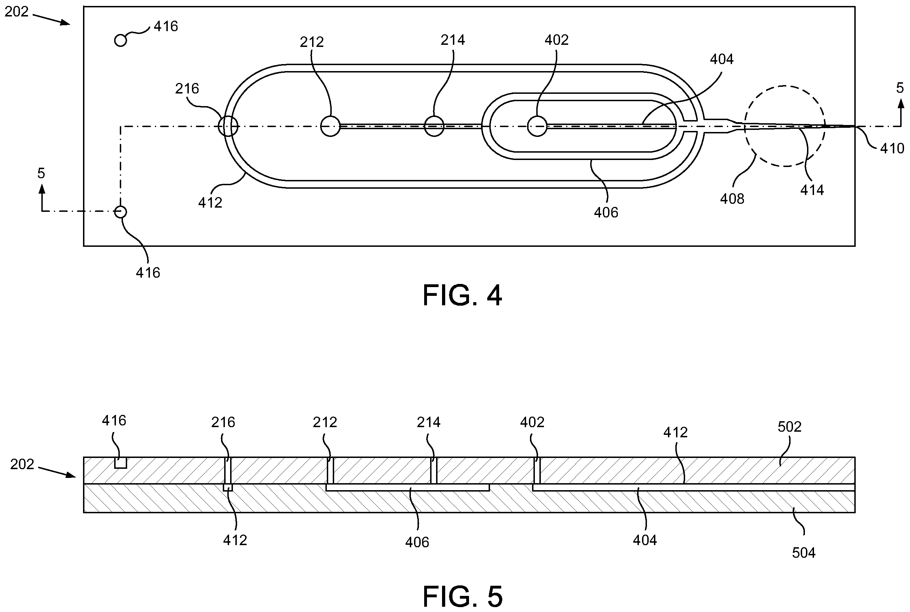

Figure 4 depicts a top view cross section of a cytometry chip 202 taken along line 4-4 of Figure 3, according to one or more embodiments of the present invention. In one embodiment, the cytometry chip 202 is formed by injection molding an industrial plastic material such as Cyclo Olefin Polymer (COP) material. The Cyclo Olefin Polymer (COP) material is transparent to facilitate optical analysis of the sample as it flows through the chip 202.

According to one embodiment of the invention, the cytometry chip 202 comprises two halves. The two halves are coupled together using, for example, the thermal bonding. Other ways of coupling the two halves will be readily apparent to one skilled in the art depending upon the material used to fabricate the chip 202.

The cytometry chip 202 includes a sheath port 212, an electrode port 214 and a vacuum port 216 as already described. The cytometry chip 202 further comprises a sample port 402, a sample channel 404, a sheath channel 406 and a vacuum channel 412. Furthermore, the cytometry chip 202 comprises an optical detection area 408 and an output port 410.

According to various embodiments, the sample port 402 is configured to accept a sample fluid that includes, for example, cells to be sorted utilizing the cytometry chip 202. The sample port 402 is coupled to the sample channel 404. The sample fluid flows through the sample channel 404 towards the output port 410 where the sample fluid is formed into droplets. The sample channel 404 has a rectangular cross section to facilitate laminar flow of the sample fluid. The dimensions of the sample channel are, for example, 100 microns by 100 microns.

Similarly, the sheath port 212 is coupled to the sheath channel 406. Sheath fluid flows through the sheath channel 406 and surrounds the sample fluid from both sides, e.g., the sheath channel 406 has an oval plan form that couples to the sample channel 404 from both sides of the sample channel 404 to facilitate a balanced coupling of the sheath fluid to the sample fluid. The sheath fluid does not mix with the sample fluid, but provides a protective layer to the sample fluid and promotes laminar flow of the fluids.

The sample fluid and the sheath fluid flow through an output channel 414 that passes through the optical detection area 408. The optics module (e.g., the optics module 108 of Figure 1) is utilized to focus the high intensity beam on the sample fluid and the sheath fluid surrounding the sample fluid. Each cell within the sample fluid is illuminated and reflects a light captured by a photo detector that is utilized to analyze the cells. The output channel 414 in the optical detection area 408 comprises a rectangular channel. In one or more embodiments, the channel 414 in the optical detection area 408 has dimensions 200 microns by 125 microns. In one embodiment, the output channel 414 has a length of, for example, 7mm with a region for optical analysis being about 5mm in length.

According to one or more embodiments, the droplets are created at the output port 410 using an acoustical transducer, as described previously. In one embodiment, the output port 410 is a circular orifice having a diameter of, for example, 100 microns. In one embodiment of the cytometry chip, the chip has a rectangular plan form with dimensions of 75mm by 25mm.

Once the sample cells are used and analysis completed, the vacuum port 216 is utilized to evacuate the sample fluid and the sheath fluid from the cytometry chip 202. The vacuum port 216 is configured to couple to a vacuum source to a vacuum channel 412 that cleans the cytometry chip 202 including the various channels such as the sample channel 404, the sheath channel 406, the output channel 414 and the output port 410. Such cleaning facilitates removal of biologically hazardous materials from the cytometry chip 202 before disposal. The vacuum channel 412 has an oval plan form that couples the vacuum port 216 to the output channel 414 from both sides of the channel 414.

To facilitate alignment of the cytometry chip 202 with the holder 204, the cytometry chip 202 may comprise at least one detent 416 or aperture. The at least one detent 416 interacts with a protrusion extending from the bottom surface of the holder.

Figure 5 depicts a cross section of the cytometry chip 202 taken along line 5-5 in Figure 4, according to one or more embodiments. The cytometry chip 202 includes a first portion 502 and a second portion 504. The first portion 502 and the second portion 504 are injection molded and then coupled together, for example, by thermally bonding the two portions together.

According to one or more embodiments, the first portion 502 comprises a top portion of the cytometry chip 202. In one embodiment, the first portion 502 is one millimeter (1 mm) thick. The first portion 502 comprises the detent 416, the sheath port 212, the electrode port 214, the vacuum port 216 and the sample port 402. The ports are centrally located along a central axis of the first portion 502. The ports are formed as apertures using a well-known injection molding technique. Other fabrication techniques such as milling may be used.

In one or more embodiments, the second portion 504 comprises a bottom portion of the cytometry chip 202. In one embodiment, the second portion 504 is one millimeter (1 mm) thick. The second portion 504 comprises various channels as described with respect to Figure 4. The channels are formed using a well-known injection molding technique. Other fabrication techniques such as milling may be used. Once assembled and bonded, each channel of the second portion 504 is coupled to a respective port formed in the first portion 502. For example, a sheath channel is coupled to the sheath port 212, the sample channel is coupled to the sample port 402, and so on.

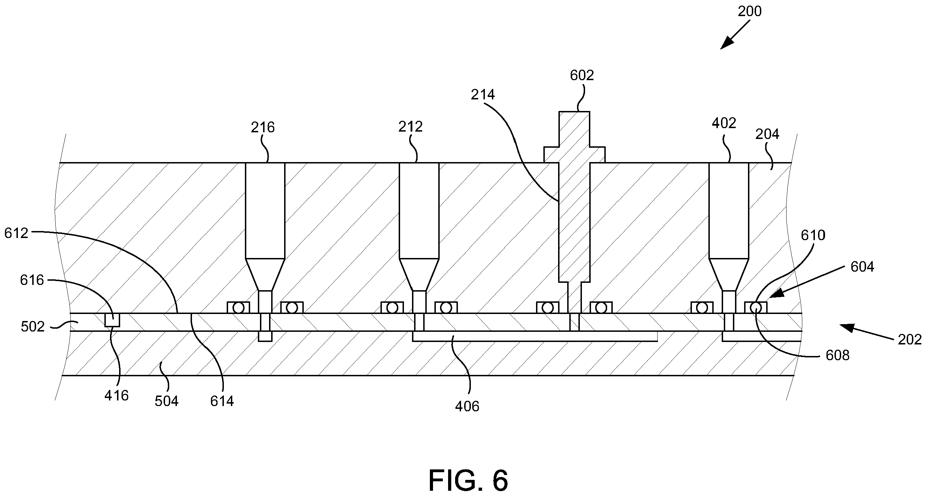

Figure 6 depicts a detailed cross section of a microfluidic assembly 200 according to one or more embodiments. As already described above, the microfluidic assembly 200 comprises a cytometry chip 202 and a chip holder 204. The chip holder 204 comprises various ports such as the sheath port 212, the electrode port 214, the vacuum port 216 and the sample port 402. Such ports are coupled to respective channels in the cytometry chip 202. To facilitate alignment of the holder 204 and the cytometry chip 202, a protrusion 616 (e.g., a pin) extends from a bottom surface 612 of the holder 204 into a detent 416 in the top surface 614 of the cytometry chip 202. The use of such an alignment means facilitates alignment of the ports passing through both the holder 204 and the cytometry chip 202. In an alternative embodiment, the protrusion is located on the top surface 614 of the cytometry chip 202 and interacts with a detent located on the bottom surface 612 of the holder 204. The various ports may be coupled to the cytometry system 100 via pressure fit couplers, through threaded fittings (not shown) and/or the like. In one or more embodiments, the electrode port 214 comprises a conductive electrode node 602.

The electrode node 602 conducts electric charge such as supplied by an electric charge source (e.g., the electric charge source 112 of Figure 1). The electrode node 602 extends through the chip holder 204 and a first portion 502 of the cytometry chip to expose a tip surface 606 of the electrode node 602 into the sheath channel 406. By applying a modulated voltage to the electrode node 602, the sheath fluid acquires a charge. The modulation frequency depends on the droplet frequency.

The chip holder 204 comprises an o-ring seals 604 circumscribing each respective port 212, 214, 216, 402. In one embodiment, each o-ring seal comprises a torus shaped gasket 608 positioned within an annular channel 610. The gasket 608 is comprised of, for example, rubber. According to various embodiments, the o-ring seals 604 are configured to circumscribe the each port at the interface with the cytometry chip 202. Upon assembling the microfluidic assembly 200, a bottom surface 612 of the holder 204 is pressure fit against a top surface 614 of the cytometry chip 202 to form the O-ring seals 604.

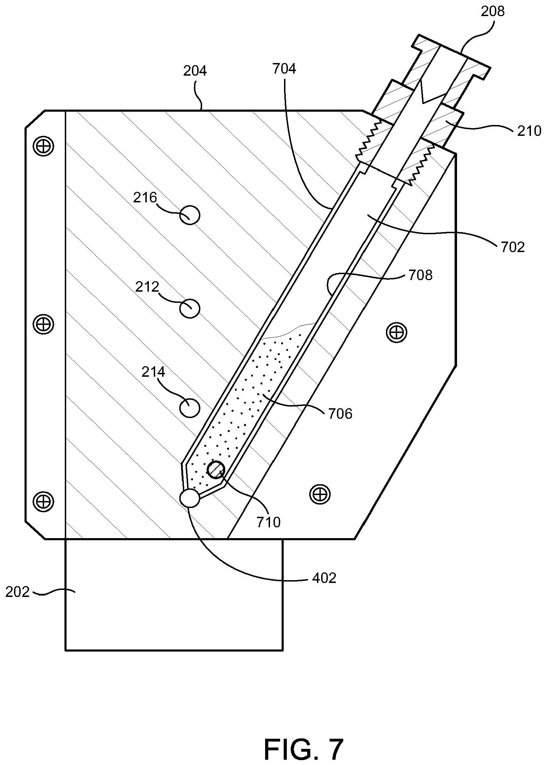

Figure 7 depicts a cross section of the chip holder 204 taken along line 7-7 of Figure 3 according to one embodiment of the invention. The chip holder 204 comprises an inlet 208, a one-way valve 210, a sample port 402 and a sample reservoir 702.

A sample 706 is injected into the sample reservoir 702, the one-way valve 210 is installed to seal the sample from the environment and the sample is stored in the sample reservoir 702 until utilized while performing cytometry. The sample inlet 208 is configured to allow the sample 706 to enter the sample reservoir 702. The inlet 208 is coupled through the one-way valve 210 to the sample reservoir 702 to ensure that a compressed gas causes the flow of the sample 706 to occur in one direction - into the sample reservoir. This arrangement prevents the sample from being "pulled back" from the reservoir 702 into the compressed gas source. Thus, the sample does not become contaminated, nor is the sample able to contaminate the environment, or the cytometry system components (e.g. the compressed gas source).

According to various embodiments, the walls 704 of the sample reservoir 702 may be treated (e.g., having a coating 708) to ensure that cells in the sample 706 do not attach to the sample reservoir walls 704. The sample reservoir 702 may comprise preservatives and/or nutrients as a coating 708 or placed into the reservoir in another manner (e.g., pellets, spheres, and the like) to maintain the sample 706. In one embodiment, the sample reservoir 702 may include one or more reagents that assist in sample processing. Such reagents may include a coating 708 and/or film on the walls 704 of the sample reservoir 702, at least one magnetic sphere 710 placed in the sample reservoir 702, at least one reagent pellet (not shown) and/or the like. In one embodiment, the at least one magnetic sphere 710 is utilized to magnetize the sample and facilitates in stem cell sorting. The inclusion of the at least one magnetic sphere 710 enables the sample holder 204 to provide a magnetization function that would otherwise have to be performed in a separate assembly prior to performing cytometry. Thus, the chip holder 204 having an integral sample reservoir can be used to remove day magnetization assembly from a stem cell sorting system.

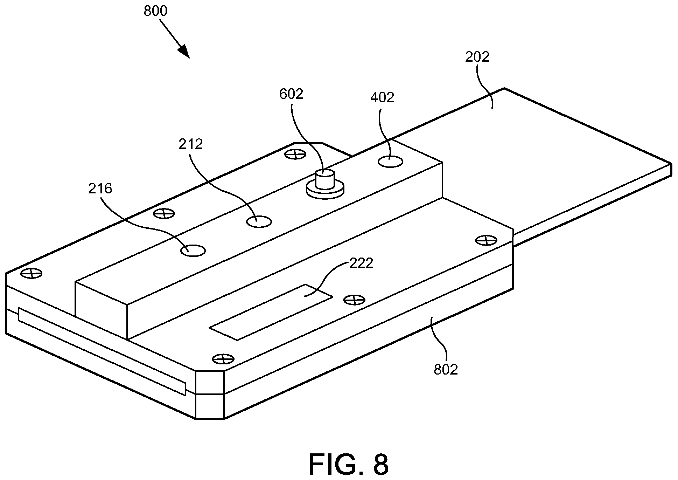

Figure 8 depicts a perspective view of a microfluidic assembly 800 according to an alternative embodiment of the invention. The microfluidic assembly 800 comprises chip holder 802 coupled to a cytometry chip 202. The chip holder 802 functions in a similar fashion to the chip holder 204. However, the chip holder 802 does not incorporate a sample reservoir into the holder. The chip holder 802 comprises various ports such as a sheath port 212, a sample port 402, a vacuum port 216 and an electrode node 602. Each of the port is configured to be coupled to a cytometry chip 202 in the same manner as described with respect to microfluidic assembly 200 in Figure 6. This version 800 of the microfluidic assembly finds use in a cytometry system 100 where the sample source (e.g., sample vessel 102 in Figure 1) is separate from the chip holder 802.

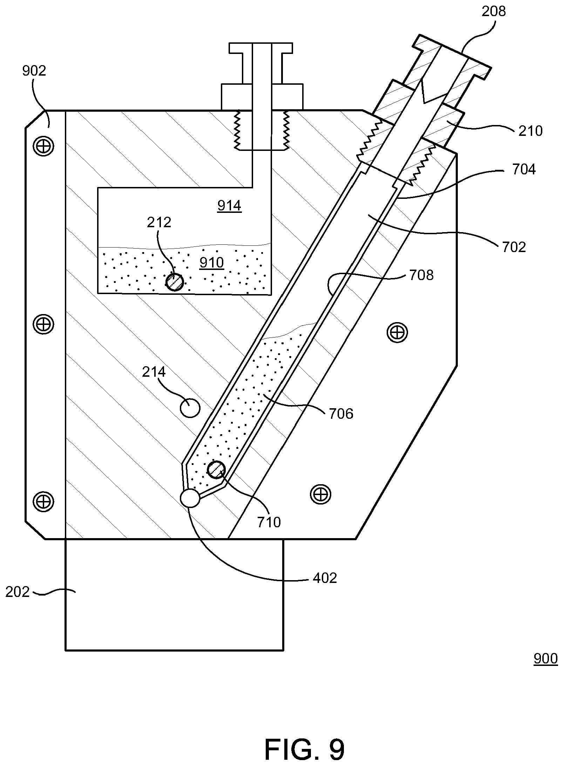

Figure 9 depicts a cross sectional view of another embodiment of microfluidic assembly 900. The microfluidic assembly 900 comprises a chip holder 902 and a cytometry chip 202. The chip holder 902 comprises, in addition to the features of the chip holder 204 of Figure 7, a sheath fluid reservoir 914 and a sheath fluid inlet 906. In one embodiment, the sheath fluid inlet 906 comprises a one-way valve. As with the sample reservoir 702, the sheath fluid 910 is supplied to the sheath fluid inlet 906, then the one-way valve is installed to retain the sheath fluid in the holder 902 and prevent contamination. A compressed gas is applied to the one-way valve 908 to pressurize the reservoir and propel the sheath fluid 910 through a sheath fluid port 212 into the cytometry chip 202. In this embodiment, the microfluidic assembly 900 provides a disposable unit that can be pre-charged with a sheath fluid 910 and/or a sample fluid 707.

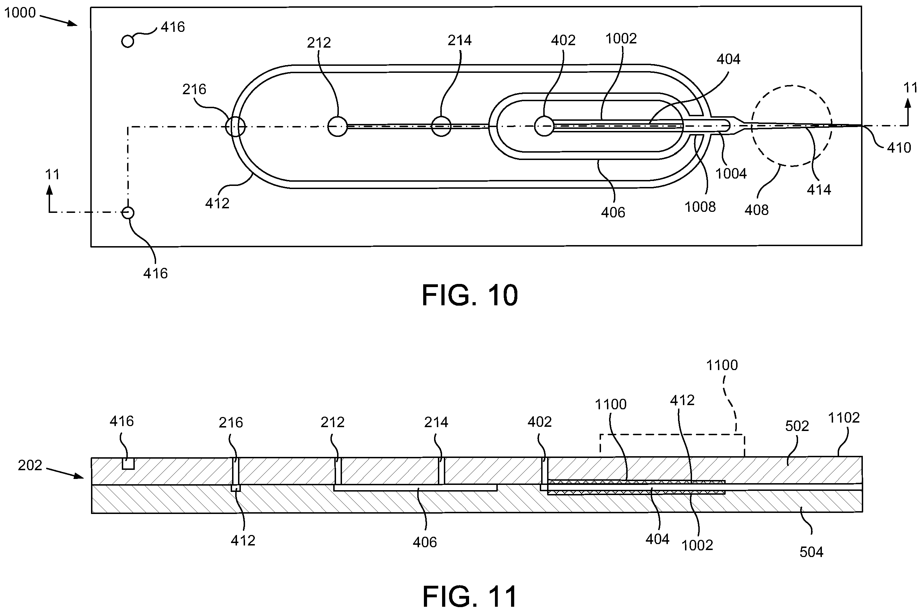

Figures 10 and 11 respectively depict a horizontal cross -sectional view and a vertical cross-sectional view of another embodiment of a cytometry chip 1000. In this embodiment, a tube 1002 is inserted into the center channel 404 extending from the sample port 402 to the junction channel 1006, where channels 406 and 412 meet the center (sample) channel 404. In one embodiment, the injection molded chip has the center channel 404 dimensioned to accept the tube 1002. The tube 1002 ends within a junction channel 1006 that is wider than the outer diameter of the tube 1002. In this manner, fluid in channel 406 can pass the end 1004 of tube 1002 into output channel 414. The tube 1002 is retained in the channel 404 when the portion 502 is positioned and adhered to portion 504. In one embodiment, portion 502 may have a channel 1100 formed therein to accommodate the tube 1002 such that the tube 1002 is retained between portion 502 and 504. In one embodiment, the tube is made of a metal such as nickel or nickel alloy. In other embodiments, the tube may be made of metal or non-metal materials.

When using a metallic tube as shown in the embodiment of Figures 10 and 11, the sample stream had a stable flow speed in the range of 1 m/s to 20 m/s. Such a cytometry cell structure promotes creation of a three-dimensional sample core stream in the output channel 414.

To create sample droplets from the stream, a thin tabular transducer 110 (e.g., 0.1 mm to 2 mm thick) may be adhered to the chip surface 1102 prior to the optical region 414. The transducer 110 may be attached by adhesive tape or an adhesive bonding material. The transducer 110 is driven with frequencies in the range 10 to 50 KHz. To protect the transducer 110 from the environment, the transducer 110 may be coated with a waterproof film.

The foregoing description, for purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the present disclosure and its practical applications, to thereby enable others skilled in the art to best utilize the invention and various embodiments with various modifications as may be suited to the particular use contemplated.

While the foregoing is directed to embodiments of the present invention, other and further embodiments of the invention may be devised without departing from the basic scope thereof, and the scope thereof is determined by the claims that follow.

## Claims

1. Apparatus for providing a sample fluid to a cytometry system (100) comprising:
a cytometry chip (202) for channeling a sample fluid from a sample fluid port (402) to an output channel (414); and
a holder (204) configured to retain the cytometry chip (202) within the holder (204), the holder (204) comprising an interface between the cytometry chip (202) and a source of the sample fluid,
wherein the holder (204) comprises a sample reservoir (702) for retaining the sample fluid, an inlet (208) configured to allow the sample to enter the sample reservoir (702), and a one-way valve (210), whereby the inlet (208) is coupled through the one-way valve (210) to the sample reservoir (702).

2. The apparatus of claim 1, wherein a wall (704) of the sample reservoir (702) is treated with a coating (708) to inhibit cell adhesion to the wall of the sample reservoir.

3. The apparatus of claim 1, wherein the cytometry chip comprises microfluidic channels and an alignment means for aligning the cytometry chip with the holder.

4. The apparatus of claim 1, wherein the interface further comprises at least one seal circumscribing at least one port.

5. The apparatus of claim 4, wherein the at least one seal comprises an annular channel and a torus shaped gasket.

6. The apparatus of claim 1, wherein the holder comprises a transducer positioned proximate a surface of the cytometry chip.

7. The apparatus of claim 1 wherein at least a portion of the channel in the cytometry chip extending from the sample fluid port to the output channel comprises a tube.

8. The apparatus of any of claims 1 to 7, wherein the holder (204) comprises at least one of a source of a sheath fluid and a source of electric charge.

9. A method of providing a sample fluid to a cytometry system comprising:
supplying a sample via a one-way valve to a sample reservoir defined within a holder wherein the holder retains a cytometry chip and comprises the one-way valve;
supplying the sample fluid from the sample reservoir of the holder to a sample fluid port of the cytometry chip;
supplying sheath fluid through a port in the holder to a sheath fluid port in the cytometry chip; and
channeling the sample fluid and the sheath fluid through the cytometry chip to an outlet channel.

10. The method of claim 9 further comprising vibrating the cytometry chip to create droplets containing sample fluid.

11. The method of claim 9 further comprising applying an electric charge to the sheath fluid through an electrode coupling electric charge from the holder to a sheath fluid channel in the cytometry chip.

12. The method of claim 9 further comprising cleaning the cytometry chip via a vacuum port extending through the holder to a vacuum channel of the cytometry chip.

13. The method of claim 9 further comprising:
supplying the sheath fluid from a sheath fluid reservoir, the sheath fluid reservoir is defined within the holder.

14. The method of claim 13 further comprising applying a compressed gas to at least one of the sample reservoir or the sheath fluid reservoir.

15. The method of claim 10 wherein at least a portion of a channel coupling the sample fluid port to the outlet channel comprises a tube.

## Patentansprüche

1. Vorrichtung zum Bereitstellen eines Probenfluids an ein Zytometriesystem (100), umfassend:
einen Zytometrie-Chip (202) zum Kanalisieren eines Probenfluids von einem Probenfluidanschluss (402) zu einem Ausgabekanal (414); und
einen Halter (204), der ausgestaltet ist, um den Zytometrie-Chip (202) innerhalb des Halters (204) festzuhalten, wobei der Halter (204) eine Schnittstelle zwischen dem Zytometrie-Chip (202) und einer Quelle des Probenfluids umfasst,
wobei der Halter (204) ein Probenreservoir (702), um das Probenfluid festzuhalten, einen Einlass (208), der ausgestaltet ist, um die Probe in das Probenreservoir (702) eintreten zu lassen, und ein Einwegeventil (210) umfasst, wobei der Einlass (208) über das Einwegeventil (210) an das Probenreservoir (702) gekoppelt ist.

2. Vorrichtung nach Anspruch 1, wobei eine Wand (704) des Probenreservoirs (702) mit einer Beschichtung (708) behandelt wird, um Zelladhäsion an der Wand des Probenreservoirs zu hemmen.

3. Vorrichtung nach Anspruch 1, wobei der Zytometrie-Chip mikrofluidische Kanäle und ein Ausrichtmittel umfasst, um den Zytometrie-Chip mit dem Halter auszurichten.

4. Vorrichtung nach Anspruch 1, wobei die Schnittstelle des Weiteren mindestens eine Dichtung umfasst, welche den mindestens einen Anschluss umgibt.

5. Vorrichtung nach Anspruch 4, wobei die mindestens eine Dichtung einen ringförmigen Kanal und einen wulstförmigen Dichtungsring umfasst.

6. Vorrichtung nach Anspruch 1, wobei der Halter einen Wandler einschließt, der in der Nähe einer Oberfläche des Zytometrie-Chips positioniert ist.

7. Vorrichtung nach Anspruch 1, wobei mindestens ein Anteil des Kanals in dem Zytometrie-Chip, der sich von dem Probenfluidanschluss zu dem Ausgabekanal erstreckt, ein Röhrchen umfasst.

8. Vorrichtung nach einem der Ansprüche 1 bis 7, wobei der Halter (204) mindestens eine von einer Quelle für ein Hüllfluid und einer Quelle für elektrische Ladung umfasst.

9. Verfahren zum Bereitstellen eines Probenfluids an ein Zytometriesystem, umfassend:
Zuführen einer Probe mittels des Einwegeventils zu einem Probenreservoir, das innerhalb eines Halters definiert ist, wobei der Halter einen Zytometrie-Chip festhält und das Einwegeventil umfasst;
Zuführen des Probenfluids aus dem Probenreservoir des Halters zu einem Probenfluidanschluss des Zytometrie-Chips;
Zuführen von Hüllfluid durch einen Anschluss in dem Halter zu einem Hüllfluidanschluss in dem Zytometrie-Chip; und
Kanalisieren des Probenfluids und des Hüllfluids durch den Zytometrie-Chip hindurch zu einem Auslasskanal.

10. Verfahren nach Anspruch 9, des Weiteren umfassend Vibrieren des Zytometrie-Chips, um Tröpfchen zu erzeugen, die Probenfluid enthalten.

11. Verfahren nach Anspruch 9, des Weiteren umfassend Anlegen einer elektrischen Ladung an das Hüllfluid durch eine Elektrode, die elektrische Ladung von dem Halter an einen Hüllfluidkanal in dem Zytometrie-Chip koppelt.

12. Verfahren nach Anspruch 9, des Weiteren umfassend Reinigen des Zytometrie-Chips mittels eines Vakuumanschlusses, der sich durch den Halter hindurch zu einem Vakuumkanal des Zytometrie-Chips erstreckt.

13. Verfahren nach Anspruch 9, des Weiteren umfassend:
Zuführen des Hüllfluids aus einem Hüllfluidreservoir, wobei das Hüllfluidreservoir in dem Halter definiert ist.

14. Verfahren nach Anspruch 13, des Weiteren umfassend Anlegen eines komprimierten Gases an mindestens eines von dem Probenreservoir oder dem Hüllfluidreservoir.

15. Verfahren nach Anspruch 10, wobei mindestens ein Anteil des Kanals, der den Probenfluidanschluss an den Auslasskanal koppelt, ein Röhrchen umfasst.

## Revendications

1. Appareil destiné à fournir un échantillon de fluide à un système de cytométrie (100) comprenant :
une puce de cytométrie (202) destinée à canaliser un échantillon de fluide depuis un orifice à échantillon de fluide (402) jusqu'à un canal de sortie (414) ; et
un support (204) configuré pour maintenir la puce de cytométrie (202) à l'intérieur du support (204), le support (204) comprenant une interface entre la puce de cytométrie (202) et une source de l'échantillon de fluide,
dans lequel le support (204) comprend un réservoir à échantillon (702) destiné à conserver l'échantillon de fluide, une entrée (208) configurée pour permettre à l'échantillon de pénétrer dans le réservoir à échantillon (702), et une vanne unidirectionnelle (210), l'entrée (208) étant couplée par la vanne unidirectionnelle (210) au réservoir à échantillon (702).

2. Appareil de la revendication 1, dans lequel une paroi (704) du réservoir à échantillon (702) est traitée avec un revêtement (708) pour empêcher l'adhérence de cellules à la paroi du réservoir à échantillon.

3. Appareil de la revendication 1, dans lequel la puce de cytométrie comprend des canaux microfluidiques et un moyen d'alignement pour aligner la puce de cytométrie avec le support.

4. Appareil de la revendication 1, dans lequel l'interface comprend en outre au moins un joint d'étanchéité encerclant au moins un orifice.

5. Appareil de la revendication 4, dans lequel l'au moins un joint d'étanchéité comprend un canal annulaire et un joint statique de forme torique.

6. Appareil de la revendication 1, dans lequel le support comprend un transducteur positionné à proximité d'une surface de la puce de cytométrie.

7. Appareil de la revendication 1 dans lequel au moins une partie du canal dans la puce de cytométrie s'étendant depuis l'orifice à échantillon de fluide jusqu'au canal de sortie comprend un tube.

8. Appareil de l'une quelconque des revendications 1 à 7, dans lequel le support (204) comprend une source d'un fluide de gaine et/ou une source de charge électrique.

9. Procédé de fourniture d'un échantillon de fluide à un système de cytométrie comprenant :
la fourniture d'un échantillon par le biais d'une vanne unidirectionnelle à un réservoir à échantillon défini à l'intérieur d'un support, le support maintenant une puce de cytométrie et comprenant la vanne unidirectionnelle ;
la fourniture de l'échantillon de fluide depuis le réservoir à échantillon du support à un orifice à échantillon de fluide de la puce de cytométrie ;
la fourniture d'un fluide de gaine par un orifice dans le support à un orifice à fluide de gaine dans la puce de cytométrie ; et
la canalisation de l'échantillon de fluide et du fluide de gaine à travers la puce de cytométrie jusqu'à un canal de sortie.

10. Procédé de la revendication 9 comprenant en outre la vibration de la puce de cytométrie pour créer des gouttelettes contenant l'échantillon de fluide.

11. Procédé de la revendication 9 comprenant en outre l'application d'une charge électrique au fluide de gaine par une électrode couplant une charge électrique provenant du support à un canal à fluide de gaine dans la puce de cytométrie.

12. Procédé de la revendication 9 comprenant en outre le nettoyage de la puce de cytométrie par le biais d'un orifice à vide s'étendant à travers le support jusqu'à un canal à vide de la puce de cytométrie.

13. Procédé de la revendication 9 comprenant en outre :
la fourniture du fluide de gaine depuis un réservoir à fluide de gaine, le réservoir à fluide de gaine étant défini à l'intérieur du support.

14. Procédé de la revendication 13 comprenant en outre l'application d'un gaz comprimé au réservoir à échantillon et/ou au réservoir à fluide de gaine.

15. Procédé de la revendication 10 dans lequel au moins une partie d'un canal couplant l'orifice à échantillon de fluide au canal de sortie comprend un tube.
